# FASCICULE DE BREVET EUROPEEN

(11) **EP 3 592 360 B1**
(45) Date de publication et mention de la délivrance du brevet: **20.04.2022**
(21) Numéro de dépôt: 18709010.5
(22) Date de dépôt: 08.03.2018
(51) Int. Cl.: A61K 31/661, A61K 9/00, A61P 25/00, A61P 25/28, A61P 21/00

(54) **ACEFAPC POUR LE TRAITEMENT DES MALADIES ACÉTYLCHOLINE DÉPENDANTES**
ACEFAPC ZUR BEHANDLUNG VON ACETYLCHOLINABHÄNGIGEN KRANKHEITEN
ACEFAPC FOR THE TREATMENT OF ACETYLCHOLINE-DEPENDENT DISEASES

(30) Priorité: 08.03.2017 FR 1751880
(43) Date de publication de la demande: 15.01.2020
(73) Titulaire: Lipther, 69100 Villeurbanne (FR); Institut National des Sciences Appliquées de Lyon, 69100 Villeurbanne (FR); Université Claude Bernard Lyon 1, 69100 Villeurbanne (FR); Institut National de la Santé et de la Recherche Médicale (INSERM), 75013 Paris (FR); Institut national de recherche pour l'agriculture, l'alimentation et l'environnement, 75007 Paris (FR)
(72) Inventeur: LAGARDE, Michel, 69150 Decines-Charpieu (FR); VERICEL, Evelyne, 69100 Villeurbanne (FR); PICQ, Madeleine, 42800 Chateauneuf (FR); GUICHARDANT, Michel, 69370 St Didier au Mont d'Or (FR); BERNOUD-HUBAC, Nathalie, 69480 Morance (FR); FOURMAUX, Baptiste, 69003 Lyon (FR)
(74) Mandataire: Be IP
(86) Numéro de dépôt international: PCT/EP2018/055706
(87) Numéro de publication internationale: WO 2018/162617

(56) Documents cités:
- FR-A1- 2 979 825
- HACHEM MAYSSA ET AL: "Efficient Docosahexaenoic Acid Uptake by the Brain from a Structured Phospholipid", MOLECULAR NEUROBIOLOGY, HUMANA PRESS, US, vol. 53, no. 5, 4 juin 2015 (2015-06-04), pages 3205-3215, XP036236430, ISSN: 0893-7648, DOI: 10.1007/S12035-015-9228-9 [extrait le 2015-06-04] cité dans la demande
- MICHEL LAGARDE ET AL: "AceDoPC, a structured phospholipid to target the brain with docosahexaenoic acid", OCL, vol. 23, no. 1, 18 décembre 2015 (2015-12-18), page D102, XP055435136, ISSN: 2272-6977, DOI: 10.1051/ocl/2015061 cité dans la demande
- NATHALIE BERNOUD-HUBAC ET AL: "Specific uptake of DHA by the brain from a structured phospholipid, AceDoPC", OCL, vol. 24, no. 2, 21 février 2017 (2017-02-21), page D205, XP055435138, ISSN: 2272-6977, DOI: 10.1051/ocl/2016053
- WILLIAM C. STANLEY ET AL: "Update on lipids and mitochondrial function : impact of dietary n-3 polyunsaturated fatty acids", CURRENT OPINION IN CLINICAL NUTRITION AND METABOLIC CARE, vol. 15, no. 2, 1 mars 2012 (2012-03-01), pages 122-126, XP055435450, US ISSN: 1363-1950, DOI: 10.1097/MCO.0b013e32834fdaf7

## Description

La présente invention concerne l'AceFaPC (1-Acetyl-2-Fattyacyl-glyceroPhosphoCholine) pour son utilisation dans la prévention et le traitement de maladies associées à une carence en acétylcholine. La présente description concerne également la molécule d'AceFaPC, pour laquelle Fa représente un acyle insaturé comprenant au moins 14 atomes de carbone, et les compositions pharmaceutiques la comprenant.

### ETAT DE LA TECHNIQUE

L'AceDoPC ou 1-acétyl,2-docosahexaénoyl-phosphatidylcholine est un transporteur d'acide docosahexaénoïque (DHA) bien connu de l'homme du métier dont la synthèse enzymatique est décrite dans la demande WO 2008/068413. Il est notamment connu comme modulateur d'activation plaquettaire par le PAF (WO2013037862A1). Il a été aussi montré que le passage d'une barrière hémato-encéphalique reconstituée est favorisé avec l'AceDoPC, par comparaison avec le DHA non-estérifié ou le PC-DHA (Hachem M. & al., Mol. Neurobiol. 2016 ; Bernoud-Hubac N. & al., OCL, 2017). Une autre étude a permis de montrer que l'AceDoPC utilisé comme transporteur de DHA dans le cerveau permettait de prévenir l'extension des lésions cérébrales lorsqu'injecté à des rats ayant subi un accident vasculaire cérébral ischémique (Chauveau et al. Curr Neurovasc Res. 2011; 8: 95-102 & Lagarde M. & al., OCL 2016, 23(1) D102). Alors que seul le traitement de l'ischémie a fait l'objet d'une étude in vivo chez un modèle de rat, l'usage de l'AceDoPC comme fournisseur de DHA au cerveau est envisagé en relation avec des maladies neurologiques associées à une carence en DHA (Hachem M. & al., Mol Neurobiol., 2016, 53(5), 3205-15). Le lien entre DHA et prévention de la maladie d'Alzheimer a été évoqué, notamment du fait que les patients atteints de la maladie d'Alzheimer présentent une carence en DHA. Ainsi un transporteur de DHA comme l'AceDoPC a pu être envisagé pour favoriser la prévention de la maladie d'Alzheimer mais pas de la traiter. Cette hypothèse faite en rapport au transport du DHA ne saurait s'étendre de manière générale à une molécule d'AceFaPc lorsque l'acide gras n'est pas le DHA.

Alors que les études se sont focalisées sur le transport et l'apport de DHA, les inventeurs ont aujourd'hui mis en évidence que l'AceDoPC et de manière générale les AceFaPC, pour lesquels Fa est un radical acyle d'acide gras insaturé d'au moins 14 atomes de carbone, peuvent rapidement transférer son groupement acétyle à un substrat comprenant un alcool, en particulier primaire.

Forts de ce constat, les inventeurs ont envisagé l'AceDoPC, non plus comme un transporteur de DHA, mais comme fournisseur d'acétyle. Puis, dans la mesure où l'AceDoPC comprend également un groupement choline, les inventeurs ont alors considéré la possibilité de produire de l'acétylcholine à partir de l'AceDoPC, dans des milieux qui sont pauvres en sources de choline et/ou d'acétyle. L'AceDoPC peut par ailleurs acétyler le thiol du coenzyme A (HSCoA) pour donner l'acétyl-CoA, précurseur de l'acétylcholine dans la réaction physiologique Acétyl-CoA + choline → acétylcholine + HSCoA.

C'est ce qui a été confirmé par diverses expérimentations qui permettent d'envisager d'utiliser l'AceDoPC et de manière plus générale les AceFaPC pour traiter les maladies associées à une carence en acétylcholine, indépendamment d'un quelconque apport en DHA.

### EXPOSE DE L'INVENTION

La présente invention concerne donc l'AceFaPC de formule générale (I) 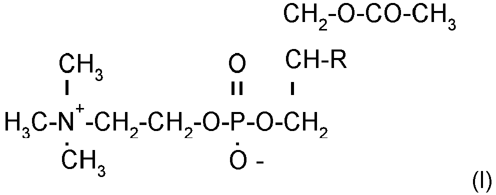 dans laquelle R représente le radical acyle d'un acide gras insaturé comprenant au moins 14 atomes de carbone, pour son utilisation comme fournisseur d'acétylcholine dans la prévention et le traitement de maladies associées à une carence en acétylcholine.

La présente description concerne également une AceFaPC de formule (I') 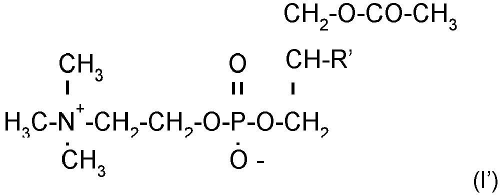 dans laquelle R' représente le radical acyle d'un acide gras insaturé comprenant au moins 14 atomes de carbone à l'exception du radical acyle du DHA, hydrates, sels pharmaceutiquement acceptables ou solvats pharmaceutiquement acceptables.

L'invention concerne également un mélange d'AceFaPC de formule (I') et d'AceDoPC, ainsi qu'une composition pharmaceutique comprenant un AceFaPC de formule (I') seul ou en mélange avec de l'AceDoPC et un excipient approprié pour son administration.

### DESCRIPTION DETAILLEE DE L'INVENTION

La présente invention concerne donc l'AceFaPC de formule générale (I) 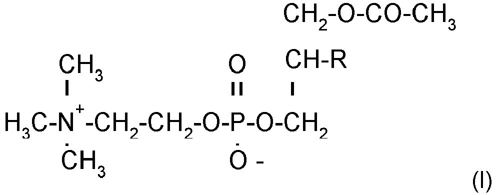 dans laquelle R représente le radical acyle d'un acide gras insaturé comprenant au moins 14 atomes de carbone, hydrates, sels pharmaceutiquement acceptables ou solvats pharmaceutiquement acceptables.

Elle concerne ces AceFaPC de formule (I) pour leur utilisation comme fournisseur d'acétylcholine dans la prévention et le traitement, plus particulièrement le traitement de maladies associées à une carence en acétylcholine.

Elle concerne également ces AceFaPC de formule (I) pour leur utilisation comme fournisseur d'acétylcholine dans une méthode de traitement ou de prévention d'une maladie associée à une carence en acétylcholine chez un patient qui comprend l'administration au dit patient d'une dose appropriée d'AceFaPC de formule (I) ou d'un mélange d'AceFaPC de formule (I).

L'invention est particulièrement adaptée pour la prévention et le traitement, plus particulièrement le traitement, de ces maladies chez l'humain. En particulier, le traitement sera employé dès lors que le patient a été identifié comme présentant une carence en acétylcholine ou encore susceptible de développer une telle carence.

Parmi les maladies associées à une carence en acétylcholine, on citera notamment
- la maladie d'Alzheimer associée à une carence en acétylcholine dans le cerveau,
- les maladies de la transmission neuromusculaire dans lesquelles une carence en acétylcholine est reconnue, notamment les maladies neuromusculaires, en particulier les myopathies avec une déficience en acétylcholine.

Par carence en acétylcholine, ou encore déficience en acétylcholine, on entend que la quantité d'acétylcholine mesurée dans un organe d'un individu est très inférieure à la normale attendue chez un individu qui n'a pas cette carence (ou individu sain). Cette baisse substantielle par rapport à un individu sain, entraine un déséquilibre ou dysfonctionnement métabolique de l'organe.

L'invention concerne donc l'AceFaPC de formule (I) ou de formule (I'), ou un mélange d'AceFaPC de formule (I), pour leur utilisation en thérapie, plus particulièrement pour le traitement de maladies associées à une carence en acétylcholine chez un patient pour lequel on a au préalable identifié la présence d'une telle carence en acétylcholine.

La présente description concerne aussi l'AceFaPC de formule (I) ou un mélange d'AceFAPC de formule (I) pour son utilisation, comme fournisseur d'acétylcholine, dans une méthode de traitement d'une maladie associée à une carence en acétylcholine chez un patient qui comprend
a) la sélection de patients chez lesquels on a identifié une carence en acétylcholine, et
b) l'administration au dit patient d'une dose appropriée d'AceFaPC de formule (I) ou (I') ou d'un mélange d'AceFaPC de formule (I).

Selon un mode préféré de réalisation de l'invention, l'AceFaPC doit être administrée de manière qu'elle soit substantiellement « intacte » lorsqu'elle atteint l'organe cible dans lequel l'acétylcholine doit être produite pour prévenir ou contrebalancer sa carence. Par substantiellement « intacte » on entend qu'une quantité suffisante d'AceFaPC atteint le dit organe sans avoir été modifiée, en particulier par hydrolyse de l'acétyle.

Il a été observé que la perte de l'acétyle était favorisée dans le tractus gastro-intestinal des mammifères (WO 2017/006047). Dès lors, les modes d'administration préférés seront ceux qui sont appropriés pour éviter le tractus gastro-intestinal. On citera en particulier les administrations par voie intraveineuse, intramusculaire, sous-cutanée, transdermique ou par inhalation.

Pour l'AceFaPC de formule (I), où R représente le radical acyle d'un acide gras insaturé comprenant au moins 14 atomes de carbone, l'acide gras insaturé comprenant au moins 14 atomes de carbone est avantageusement un acide gras de plus de 18 atomes de carbone pouvant aller jusqu'à plus de 22 atomes de carbone, en particulier 16 18, 20, 22 et 24 atomes de carbone. Ces acides gras insaturés sont préférentiellement polyinsaturés. Ces acides gras insaturés sont bien connus de l'homme du métier.

Ils sont particulièrement choisis parmi les acides palmitoléique, oléique, linoléique (LA), alpha- ou gamma-linolénique (ALA ou GLA), arachidonique (ARA), adrénique (AdA) eicosapentaénoique (EPA), dihomo-gamma-linolénique, docosapentaénoïque (DPA) docosahexaénoïque (DHA), érucique et nervonique.

De manière préférée, le radical R de l'AceFaPC de formule (I) est le radical acyle d'un acide gras polyinsaturé choisi parmi les acides oléique (OL), linoléique (LA), alpha- ou gamma-linolénique (ALA ou GLA), arachidonique (ARA), eicosapentaénoique (EPA) et docosahexaénoïque (DHA). De préférence, le R de l'AceFaPc de formule (I) est le radical acyle d'un acide gras polyinsaturé choisi parmi l'acide arachidonique (ARA), et l'acide docosahexaénoïque (DHA). Ces produits préférés sont appelés AceArPC et AceDoPC, respectivement.

Pour l'AceFaPC de formule I', R' est avantageusement choisi parmi les acyles des acides gras définis ci-dessus à l'exception du DHA.

Par « hydrates », on entend un composé sous forme hydratée. On peut citer, à titre d'exemple, les semi-hydrates, monohydrates et polyhydrates.

Les sels des composés de formule (I) ou de formule (I') selon la présente invention comprennent ceux avec des acides ou des bases, en fonction des substituants présents. On peut citer les sels pharmaceutlquement acceptables, tels que des sels de sodium, de potassium, de calcium.

Par « solvats », on entend une forme du composé associé à un ou plusieurs molécules de solvant, notamment utilisé lors de sa synthèse ou lors de sa purification, sans pour autant être en solution dans ce dernier. Le solvant en question sera pharmaceutiquement acceptable.

Par dose appropriée, ou quantité appropriée, on entend selon l'invention toute quantité qui permet d'augmenter la quantité d'acétylcholine et de préférence de rétablir une quantité d'acétylcholine proche de la normale attendue chez un individu sain. Cette dose appropriée peut être prise en une ou plusieurs fois, avec des prises répétées dans le temps. Dans la mesure où la maladie traitée est une affection chronique, le traitement pourra être pris toute la vie du patient, les doses appropriées étant adaptées en fonction de l'évolution de la maladie.

Pour la prévention et le traitement de maladies associées à une carence en acétylcholine, l'homme du métier pourra choisir d'employer un AceFaPC seul ou un mélange d'AceFaPC. Il pourra également combiner l'AceFaPC ou le mélange d'AceFaPC avec les traitements usuels des maladies associées à une carence en acétylcholine.

L'invention concerne également un mélange d'AceFaPC de formule (I) définie précédemment comprenant au moins deux AceFaPC pour lesquelles les radicaux R sont différents, en toutes proportions. Selon un mode préféré de réalisation de l'invention, au moins l'une des AceFaPC du mélange est l'AceDoPC, molécule de formule I pour laquelle R est le radical acyle du DHA. Un mélange préféré selon l'invention est donc un mélange comprenant de l'AceDoPC et au moins un AceFaPC de formule (I') dans laquelle R est le radical acyle d'un acide gras polyinsaturé choisi parmi les acides oléique (OL), linoléique (LA), alpha- ou gamma-linolénique (ALA ou GLA), arachidonique (ARA) et, eicosapentaénoique (EPA), de préférence ARA, en toutes proportions.

La préparation des AceFaPC est connue de l'homme du métier, notamment suivant la méthode décrite dans les demandes WO 2008/068413 ou WO 2017/006047. Les mélanges d'AceFaPC selon l'invention peuvent être préparés en mélangeant deux AceFaPC purifiées, ou bien en préparant les AceFaPC à partir d'une source de phosphatidylcholines insaturées comprenant un mélange d'acides gras insaturés, par exemple un mélange de DHA et d'ARA dans des proportions prédéterminées.

Le mélange d'AceFaPC selon l'invention peut comprendre plus de deux AceFaPC différentes, notamment lorsque la source de phosphatidylcholines insaturés comprend un un mélange de plus de deux acides gras insaturés.

Les sources d'acides gras insaturés utiles pour la préparation des AceFaPC selon l'invention, et en particulier les mélanges d'AceFaPC, sont bien connues de l'homme du métier. On peut donner l'exemple des phosphatidylcholines de jaune d'œuf comprenant en position sn-2 60% d'oléoyle (18 :1), 30% de linoléoyle (18 :2), 8% d'arachidonoyle (20 :4) et 2% de docosahexaénoyle (22 :6) (https://kewpie.co.jp).

Avantageusement, le rapport pondéral de la première AceFaPC à la deuxième AcFaPC va de 1/99 à 99/1. Notamment lorsque la première AceFaPC est l'AceDoPC, la teneur en AceDoPC dans le mélange peut aller de 1 à 10% en poids, voire plus, la teneur en AceDoPC dans le mélange pouvant dépendre à la fois de la source de phosphatidylcholines insaturées et de sa teneur en DHA, et d'éventuelles étapes de purifications et de concentration du mélange.

On aura avantageusement, dans un mélange d'AceFaPC selon l'invention, les proportions relatives en acyles d'acides gras suivantes

| Acyle | % relatif |
|---|---|
| Oléyle | 10-80 |
| Linoléoyle | 5-50 |
| Linolénoyle | 0-5 |
| Arachidonoyle | 0-10 |
| Eicosapentaénoyle | 0-10 |
| Docosahexaénoyle | 0-10 |

Selon un mode particulier de réalisation de l'invention, la teneur relative en docosaheaxanoyle dans ce mélange peut aller de 1 à 10%.

L'invention concerne aussi un produit de combinaison, ou « kit of parts », pour une utilisation simultanée ou décalée dans le temps, qui comprend d'une part de l'AceDoPC et d'autre part au moins une AceFaPC de formule (I) qui n'est pas de l'AceDoPC, telle que définie précédemment.

L'invention concerne également une composition pharmaceutique comprenant au moins un mélange d'AceDoPC et d'au moins une AceFaPC de formule (I') (AceFaPC de formule (I) qui n'est pas l'AceDoPC) et au moins un excipient pharmaceutiquement acceptable.

L'homme du métier connaît bien les excipients pharmaceutiquement acceptables susceptibles d'être employés pour la préparation d'une composition pharmaceutique, notamment décrits dans les ouvrages de référence de la pharmacopée. Il choisira de préférence les excipients qui vont préserver la structure des AceFaPC pour leur conservation, en particulier pour prévenir l'hydrolyse de la position sn-1 entrainant la perte de l'acétyle.

Les compositions pharmaceutiques sont de préférence sous une forme appropriée pour une administration par voie intraveineuse, intramusculaire, sous-cutanée, transdermale ou par inhalation.

### DESCRIPTION DES FIGURES

La Figure 1 décrit la synthèse d'acétylcholine à partir d'AceFaPC sous l'action d'une phospholipase D.
La Figure 2 montre la détection de l'acétylcholine et de son produit de fragmentation principal par spectrométrie de masse.
La Figure 3 montre les pourcentages de passage du sang au cerveau (rat) sur la base du DHA radioactif, en fonction du temps. *(*Hachem et al. Mol. Neurobiol. 2016*)*

### EXEMPLES

Plusieurs expériences ont été conduites à partir d'AceFaPC (DHA et acide oléique) comme précurseur possible d'acétylcholine et mesure finale de cette dernière par radiochromatographie ou spectrométrie de masse.

### Exemple 1 : Synthèse d'acétylcholine à partir d'AceFaPC avec un homogénat de cerveau de rat

Un homogénat de cerveau de rat a été incubé avec de l'AceFaPC, marqué au ¹⁴C sur l'acétyle, dans un tampon Tris-HCI pH 8 en présence d'un cocktail d'anti-protéases et d'un inhibiteur d'acétylcholine estérase. L'incubation se fait à 37°C pendant une heure. Après extraction par un mélange éthanol/chloroforme, la phase organique et la phase aqueuse sont séparées et analysées. Après séparation par chromatographie sur couche mince, les produits sont visualisés avec un lecteur de radioactivité. Une tache radioactive correspondant à la migration de l'acétylcholine est détectée. L'incubation d'un homogénat de cerveau de rat avec de l'AceFaPC marqué permet donc la synthèse d'acétylcholine radioactive, après libération de choline par la phospholipase D cérébrale et couplage chimique de cette choline avec un groupement acétyle marqué en position sn-1 de l'AceFaPC et/ou couplage de l'acétyle radioactif apporté par AceFaPC et la choline endogène.

### Exemple 2 : Synthèse d'acétylcholine à partir d'AceFaPC avec une phospholipase D

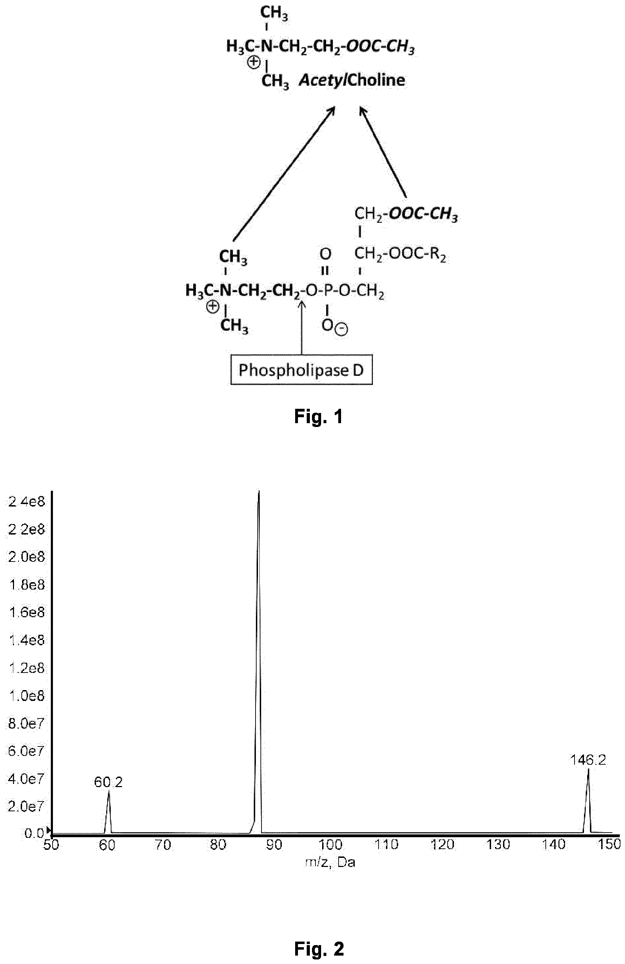

L'incubation acellulaire d'AceFaPC a été faite en présence d'une phospholipase D d'origine microbienne (de *Streptomyces chromofuscus)* dans un tampon Tris-HCI pH 8 (pH cérébral) pendant une heure à 37°C. A la suite de l'incubation, la phospholipase D a été détruite par ajout d'éthanol. Après centrifugation, le mélange aqueux éthanolique a été séparé et évaporé à sec. Le résidu a été remis en solution dans le mélange 95% d'acétonitrile / 5 % de formiate d'ammonium puis filtré par centrifugation. La mise en évidence d'acétylcholine dans la solution a été faite par spectrométrie de masse. L'acétylcholine a été détectée à la fois sur la base de sa masse moléculaire (146) et de celle de son produit de fragmentation principal (87 : ion majoritaire correspondant au radical CH₃-COO-CH₂-CH₂-) (Figure 2).

Dans ces expériences acellulaires, deux substrats ont été utilisés en considérant la plus faible insaturation pour l'acyle en position sn-2, soit le radical oléoyle (R₂-COO-, Fig 1) : AceOIPC et la plus forte avec le radical docosahexaénoyle : AceDoPC^{®}. Pour AceOIPC, environ 5% de la choline libérée a été convertie en acétylcholine. Pour AceDoPC^{®}, cette conversion a atteint 36%. Les raisons de cette différence sont inconnues à ce stade de l'expérimentation. On peut toutefois émettre l'hypothèse que la conjugaison de la choline avec le radical acétyle est meilleure si la position sn-2 est moins encombrée, ce qui est le cas pour le radical docosahexaénoyle (avec 6 double liaisons) comparativement au radical oléoyle (avec une seule double-liaison) car le repliement de la chaîne acyle avec 6 double liaisons est beaucoup plus important que pour une seule double liaison. Ce résultat est particulièrement encourageant pour AceFaPC contenant des acyles polyinsaturés, comme docosahexaénoyle et arachidonoyle (non encore testé), les deux acyles les plus importants du cerveau.

L'incubation acellulaire d'AceFaPC a également été faite en présence d'une quantité équimoléculaire de chlorure de choline dissout dans un tampon Tris-HCI pH 8 pendant une heure à 37°C sous agitation. Après évaporation à sec sous azote, le résidu a été mis en solution dans le mélange 95% d'acétonitrile / 5 % de formiate d'ammonium. Comme précédemment décrit, après filtration par centrifugation, la mesure d'acétylcholine a été faite par spectrométrie de masse.

Ces expériences montrent que les incubations d'AceFaPC en présence de phospholipase D (qui libère la choline de l'AceFaPC) ou en présence de choline exogène permettent la synthèse d'acétylcholine.

La proximité des deux groupements constitutifs (acétyle et choline) de l'acétylcholine sur la même molécule, distants d'un nm environ, est de nature à faciliter leur conjugaison en produit final par rapport à la distance au sein d'une même cellule (distance de l'ordre du µm) comme il est admis dans tous les processus biochimiques concernés par cette proximité des réactants.

Cette proximité est résumée par la réaction figurant dans la Figure 1.

Les résultats confirment l'hypothèse du mécanisme d'action de transformation de l'AceFaPC en acétylcholine dans le cerveau sous l'action de la phospholipase D cérébrale.

### Exemple 3 : Transport de l'AceDoPC dans le cerveau de rats (Hachem et al. 2016)

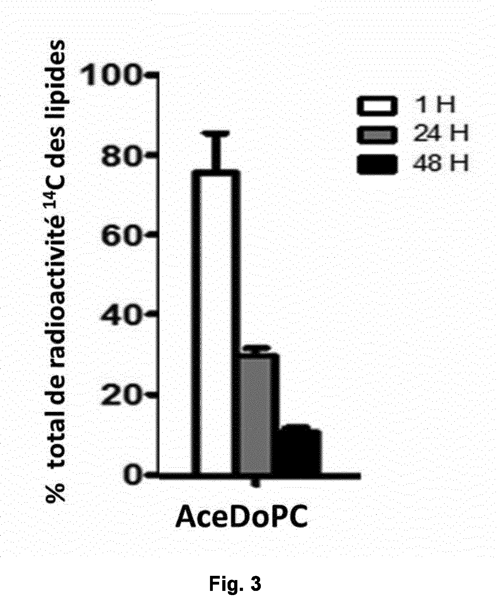

De l'AceDoPC marqué au ¹⁴C sur le résidu docosahexaénoyle a été injecté dans la circulation sanguine de différents rats. Après 1h, 24h et 48h, les cerveaux des rats ont été analysés pour localiser la radioactivité dans différents compartiments lipidiques. Pour ce qui concerne l'AceDoPC, sa radioactivité passait de 80% de la totalité injectée après 1h, puis 30% après 24h et 10% après 48h (Figure 3). Ces résultats confirment que l'AceDoPC injecté dans la circulation sanguine est substantiellement « intacte » lorsqu'elle atteint le cerveau où il est métabolisé, notamment par la perte de son résidu acétyle avec la formation d'acétylcholine.

### REFERENCES

- WO2013037862
- WO 2008/068413
- Bernoud-Hubac N. & al., OCL 2017, 24(2) D205
- Hachem M. & al., Mol Neurobiol., 2016, 53(5), 3205-15
- Lagarde M. & al., OCL 2016, 23(1) D102

## Revendications

1. AceFaPC de formule générale (I) 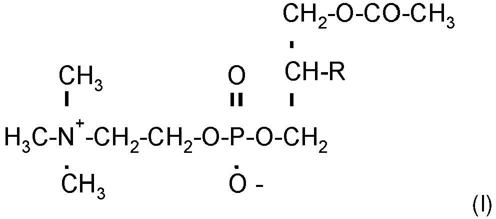
dans laquelle R représente le radical acyle d'un acide gras insaturé comprenant au moins 14 atomes de carbone, hydrates, sels pharmaceutiquement acceptables ou solvats pharmaceutiquement acceptables,
pour son utilisation comme fournisseur d'acétylcholine dans la prévention et le traitement de maladies associées à une carence en acétylcholine, lesdites maladies étant choisies parmi la maladie d'Alzheimer et les maladies neuro-musculaires.

2. AceFaPC pour son utilisation selon la revendication 1, **caractérisée en ce qu'**elle est administrée par une voie appropriée pour éviter le tractus gastro-intestinal.

3. AceFaPC pour son utilisation selon la revendication 2, **caractérisé en ce qu'**elle est administrée par voie intraveineuse, intramusculaire, sous-cutanée, transdermique ou par inhalation.

4. AceFaPC pour son utilisation selon l'une des revendications 1 à 3, **caractérisé en ce que** R représente le radical acyle d'un acide gras insaturé choisi parmi les acides oléique (OA), linoléique (LA), alpha- ou gamma-linolénique (ALA ou GLA), eicosapentaénoique (EPA), et arachidonique (ARA).

5. Mélange d'AceFaPC de formule (I) telle que définie à la revendication 1, **caractérisé en ce qu'**il comprend une première AceFaPC de formule (I) dans laquelle R est un premier radical acyle d'un acide gras insaturé comprenant au moins 14 atomes de carbone et au moins une deuxième AceFaPC de formule (I) dans laquelle R représente un radical acyle d'un acide gras insaturé comprenant au moins 14 atomes différent du radical acyle de la première AceFaPC.

6. Mélange d'AceFaPC selon la revendication 5, **caractérisé en ce qu'**il comprend une première AceFaPC de formule (I) dans laquelle R est le radical acyle du DHA (AceDoPC) et au moins une AceFaPC de formule (I) dans laquelle R représente un radical acyle d'un acide gras insaturé comprenant au moins 14 atomes de carbone et n'est pas le radical acyle du DHA.

7. Mélange selon la revendication 5, **caractérisée en ce que** l'acide gras insaturé de la deuxième AceFaPC de formule (I) est choisi parmi les acides oléique (OA), linoléique (LA), alpha- ou gamma-linolénique (ALA ou GLA), eicosapentaénoique (EPA), et arachidonique (ARA).

8. Mélange d'AceFaPC selon la revendication 7, **caractérisé en ce que** l'acide gras insaturé de la deuxième AceFaPC est l'acide arachidonique (ARA).

9. Mélange d'AceFaPC selon la revendication 5, **caractérisé en ce que** les proportions relatives en acyles d'acides gras dans les différentes AceFaPC de formule (I) sont les suivantes
| **Acyle** | **% relatif** |
|---|---|
| Oléyle | 10-80 |
| Linoléoyle | 5-50 |
| Linolénoyle | 0-5 |
| Arachidonoyle | 0-10 |
| Eicosapentaénoyle | 0-10 |
| Docosahexaénoyle | 0-10 |

10. Mélange d'AceFaPC selon l'une des revendications 5 à 9 pour son utilisation comme fournisseur d'acétylcholine dans la prévention et le traitement de maladies associées à une carence en acétylcholine, lesdites maladies étant choisies parmi la maladie d'Alzheimer et les maladies neuro-musculaires.

11. Produit de combinaison, pour une utilisation simultanée ou décalée dans le temps, **caractérisé en ce qu'**il comprend d'une part de l'AceDoPC et d'autre part au moins une AceFaPC de formule (I) telle que définie à la revendication 1 dans laquelle R représente un radical acyle d'un acide gras insaturé comprenant au moins 14 atomes de carbone et R n'est pas le radical acyle du DHA.

12. Composition pharmaceutique, **caractérisée en ce qu'**elle comprend un mélange d'AceFaPC selon l'une des revendications 5 à 9 et au moins un excipient pharmaceutiquement acceptable.

13. Composition selon la revendication 12, **caractérisée en ce qu'**elle est sous une forme appropriée pour une administration par voie intraveineuse, intramusculaire, sous-cutanée, transdermique ou par inhalation.

## Patentansprüche

1. AceFaPC mit der folgenden allgemeinen Formel (I) 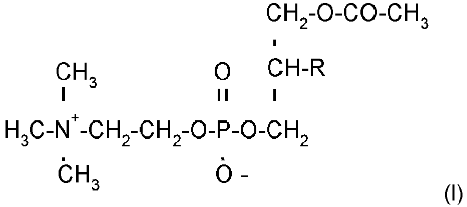
wobei R das Acylradikal einer ungesättigten Fettsäure darstellt, das mindestens 14 Kohlenstoffatome, Hydrate, pharmazeutisch annehmbare Salze oder pharmazeutisch annehmbare Solvate umfasst,
für seine Verwendung als Lieferant von Acetylcholin bei der Prävention und der Behandlung von Erkrankungen in Verbindung mit einem Acetylcholinmangel, wobei die Erkrankungen aus der Alzheimerkrankheit und den neuromuskulären Erkrankungen ausgewählt ist.

2. AceFaPC zur Verwendung nach Anspruch 1, **dadurch gekennzeichnet, dass** es auf einem Weg verabreicht wird, der geeignet ist, den Verdauungstrakt zu meiden.

3. AceFaPC zur Verwendung nach Anspruch 2, **dadurch gekennzeichnet, dass** es auf intravenösem, intramuskulärem, subkutanem, transdermalem Weg oder durch Inhalation verabreicht wird.

4. AceFaPC zur Verwendung nach einem der Ansprüche 1 bis 3, **dadurch gekennzeichnet, dass** R das Acylradikal einer ungesättigten Fettsäure darstellt, die aus Öl- (OA), Linol-(LA), Alpha- oder Gamma-Linolen- (ALA oder GLA), Eicosapentaen- (EPA) und Arachidonsäure (ARA) ausgewählt ist.

5. AceFaPC-Gemisch mit der Formel (I) nach Anspruch 1, **dadurch gekennzeichnet, dass** es ein erstes AceFaPC mit der Formel (I), wobei R ein erstes Acylradikal einer ungesättigten Fettsäure ist, das mindestens 14 Kohlenstoffatome umfasst, und mindestens ein zweites AceFaPC mit der Formel (I) umfasst, in der R ein Acylradikal einer ungesättigten Fettsäure darstellt, das mindestens 14 Atome umfasst, die sich von dem Acylradikal des ersten AceFaPC unterscheiden.

6. AceFaPC-Gemisch nach Anspruch 5, **dadurch gekennzeichnet, dass** es ein erstes AceFaPC mit der Formel (I), wobei R das Acylradikal des DHA (AceDoPC) ist, und mindestens ein AceFaPC mit der Formel (I) umfasst, wobei R ein Acylradikal einer ungesättigten Fettsäure darstellt, das mindestens 14 Kohlenstoffatome umfasst und nicht das Acylradikal des DHA ist.

7. Gemisch nach Anspruch 5, **dadurch gekennzeichnet, dass** die ungesättigte Fettsäure des zweiten AceFaPC mit der Formel (I) aus Öl- (OA), Linol- (LA), Alpha- oder Gamma-Linolen- (ALA oder GLA), Eicosapentaen- (EPA) und Arachidonsäure (ARA) ausgewählt ist.

8. AceFaPC-Gemisch nach Anspruch 7, **dadurch gekennzeichnet, dass** die ungesättigte Fettsäure des zweiten AceFaPC die Arachidonsäure (ARA) ist.

9. AceFaPC-Gemisch nach Anspruch 5, **dadurch gekennzeichnet, dass** die entsprechenden Anteile an Fettsäureacylen in den verschiedenen AceFaPC mit der Formel (I) die folgenden sind:
| **Acyl** | **Anteil %** |
|---|---|
| Oleyl | 10 bis 80 |
| Linoleoyl | 5 bis 50 |
| Linolenoyl | 0 bis 5 |
| Arachidonoyl | 0 bis 10 |
| Eicosapentaenoyl | 0 bis 10 |
| Docosahexaenoyl | 0 bis 10 |

10. AceFaPC-Gemisch nach einem der Ansprüche 5 bis 9 zur Verwendung als Lieferant von Acetylcholin bei der Prävention und der Behandlung von Erkrankungen in Verbindung mit einem Acetylcholinmangel, wobei die Erkrankungen aus der Alzheimerkrankheit und den neuromuskulären Erkrankungen ausgewählt sind.

11. Kombinationsprodukt zur gleichzeitigen oder zeitlich versetzten Verabreichung, **dadurch gekennzeichnet, dass** es einerseits AceDoPC und andererseits mindestens ein AceFaPC mit der Formel (I) nach Anspruch 1 umfasst, wobei R ein Acylradikal einer ungesättigten Fettsäure darstellt, das mindestens 14 Kohlenstoffatome umfasst, und R nicht das Acylradikal des DHA ist.

12. Pharmazeutische Zusammensetzung, **dadurch gekennzeichnet, dass** sie das AceFaPC-Gemisch nach einem der Ansprüche 5 bis 9 und mindestens einen pharmazeutisch annehmbaren Hilfsstoff umfasst.

13. Zusammensetzung nach Anspruch 12, **dadurch gekennzeichnet, dass** sie in einer Form vorliegt, die sich zur intravenösen, intramuskulären, subkutanen, transdermalen Verabreichung oder durch Inhalation eignet.

## Claims

1. An AceFaPC of general formula (I) 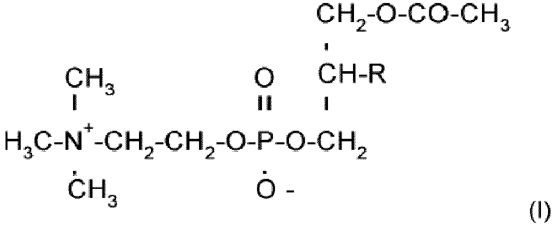
wherein R represents the acyl radical of an unsaturated fatty acid comprising at least 14 carbon atoms, hydrates, pharmaceutically acceptable salts or pharmaceutically acceptable solvates,
for use as a supplier of acetylcholine in the prevention and treatment of diseases associated with acetylcholine deficiency, said diseases being selected from Alzheimer's disease and neuromuscular diseases.

2. The AceFaPC for use according to claim 1, **characterized in that** it is administered by an appropriate route to avoid the gastrointestinal tract.

3. The AceFaPC for use according to claim 2, **characterized in that** it is administered by intravenous, intramuscular, subcutaneous, transdermal route or by inhalation.

4. The AceFaPC for use according to one of claims 1 to 3, **characterized in that** R represents the acyl radical of an unsaturated fatty acid selected from oleic (OA), linoleic (LA), alpha- or gamma-linolenic (ALA or GLA), eicosapentaenoic (EPA), and arachidonic (ARA) acids.

5. A mixture of AceFaPC of formula (I) as defined in claim 1, **characterized in that** it comprises a first AceFaPC of formula (I) wherein R is a first acyl radical of an unsaturated fatty acid comprising at least 14 carbon atoms and at least a second AceFaPC of formula (I) wherein R represents an acyl radical of an unsaturated fatty acid comprising at least 14 atoms different from the acyl radical of the first AceFaPC.

6. The AceFaPC mixture according to claim 5, **characterized in that** it comprises a first AceFaPC of formula (I) wherein R is the acyl radical of DHA (AceDoPC) and at least one AceFaPC of formula (I) wherein R represents an acyl radical of an unsaturated fatty acid comprising at least 14 carbon atoms and is not the acyl radical of DHA.

7. The mixture according to claim 5, **characterized in that** the unsaturated fatty acid of the second AceFaPC of formula (I) is selected from oleic (OA), linoleic (LA), alpha- or gamma-linolenic (ALA or GLA), eicosapentaenoic (EPA) and arachidonic (ARA) acids.

8. The AceFaPC mixture according to claim 7, **characterized in that** the unsaturated fatty acid of the second AceFaPC is arachidonic acid (ARA).

9. The AceFaPC mixture according to claim 5, **characterized in that** the relative proportions of fatty acid acyls in the different AceFaPCs of formula (I) are as follows:
| **Acyl** | **relative %** |
|---|---|
| Oleyl | 10-80 |
| Linoleoyl | 5-50 |
| Linolenoyl | 0-5 |
| Arachidonoyl | 0-10 |
| Eicosapentaenoyl | 0-10 |
| Docosahexaenoyl | 0-10 |

10. The AceFaPC mixture according to one of claims 5 to 9 for use as a supplier of acetylcholine in the prevention and treatment of diseases associated with acetylcholine deficiency, said diseases being selected from Alzheimer's disease and neuromuscular diseases.

11. A combination product, for simultaneous or staggered use over time, **characterized in that** it comprises on the one hand AceDoPC and on the other hand at least one AceFaPC of formula (I) as defined in claim 1 wherein R represents an acyl radical of an unsaturated fatty acid comprising at least 14 carbon atoms and R is not the acyl radical of DHA.

12. A pharmaceutical composition, **characterized in that** it comprises a mixture of AceFaPC according to one of claims 5 to 9 and at least one pharmaceutically acceptable excipient.

13. The composition according to claim 12, **characterized in that** it is in a form suitable for administration by intravenous, intramuscular, subcutaneous, transdermal route or by inhalation.
